# EUROPÄISCHE PATENTANMELDUNG

(11) **EP 3 095 905 A2**
(43) Veröffentlichungstag der Anmeldung: **23.11.2016**
(21) Anmeldenummer: 16162594.2
(22) Anmeldetag: 29.03.2016
(51) Int. Cl.: D04C 1/08, A61F 2/00

(54) **TEXTILES IMPLANTAT**

(30) Priorität: 30.03.2015 DE 102015104884
(71) Anmelder: SERAG-WIESSNER GmbH & Co. KG, 95119 Naila (DE)
(72) Erfinder: Gramalla, Oliver, 95152 Selbitz (DE)
(74) Vertreter: Beckord & Niedlich

(57) **Zusammenfassung**

Die Erfindung betrifft ein textiles, ein Netz mit Maschen umfassendes und einen nahtlosen Schlauch bildendes Implantat zur Ummantelung zumindest eines Teils eines Organs (K), wobei das Netz knotenlos, insbesondere gewirkt, gewebt oder gelegt ist und wobei der Schlauch zur radialen Anpassung an den Teil des Organs kontrahierbar ist.

## Beschreibung

Die Erfindung betrifft ein textiles Implantat aus einem netzartigen Material, das zum Unterstützen oder Abstützen von organischen Strukturen im menschlichen oder tierischen Körper einsetzbar ist.

Die DE 10 2004 025 403 beschreibt und zeigt ein implantierbares textiles Flächengebilde, das durch Klöppeln ausgebildet ist, wodurch wünschenswerte Eigenschaften eines Implantats, insbesondere zum Unterstützen von Körperstrukturen erreicht werden.

Aufgabe der Erfindung ist es, ein Implantat anzugeben, das sich minimalinvasiv einführen und dann intracorporal ohne weitere Hilfsinstrumente individuell anpassen lässt.

Diese Aufgabe wird durch ein textiles, ein Netz mit Maschen bildendes schlauchförmiges Implantat nach Anspruch 1 gelöst. Textil bedeutet, dass es sich bei dem Material des Implantats um ein textiles Gebilde aus Fasern bzw. aus daraus hergestellten linienförmigen textilen Gebilden wie z. B. Filamenten, Garnen oder Zwirnen handelt, die zu Geweben, Gewirken, Nähgewirken, Gestricken, Geflechten, Vliesstoffen oder Filzen verarbeitet sind. Das Implantat unterscheidet sich damit insbesondere von weitgehend formstabilen rohrförmigen Implantaten, insbesondere den Gefäßstützen oder Stents, aus verhältnismäßig steifen Netzen, insbesondere aus metallischen Materialien, die einem umfangsseitigen Druck von außen widerstehen müssen. Denn im Gegensatz zu den Stents lassen sich die erfindungsgemäßen schlauchförmigen Implantate an Körperstrukturen von deren Außenseite aus flexibel anpassen, um sich an sie anzuschmiegen, sie zu halten und zu stützen. Dafür und für eine erfolgreiche Einheilung verfügen sie aufgrund ihrer textilen Struktur über eine gewisse Elastizität und Weichheit bis hin zu einem maximalen Radius. Im Einsatz unterliegen sie also einer Druckbelastung von innen bzw. bringen eine nach innen gerichtete Druckkraft auf. Ihr textiles Material hat daher einer Zugbelastung zu widerstehen, sie sind also zugstabil. Stents dagegen müssen im Einsatz einer Druckbelastung von außen widerstehen bzw. eine nach außen gerichtete Druckkraft aufbringen, sind also druckstabil.

Als schlauchförmiges Implantat verfügt es über eine Mantelfläche. Sie wird durch ein Netz gebildet, also durch ein textiles, in einer Abwicklung der Mantelfläche flächiges Gebilde mit regelmäßigen Öffnungen bzw. Maschen. Die Maschen können beispielsweise rhombisch, quadratisch, oval oder sechseckig ausgebildet sein. Der Begriff Masche ist dabei nicht in dem engeren Sinne einer Fadenschlinge zu verstehen, die in andere Fadenschlingen eingehängt ist, wodurch Strick- oder Wirkbaren entstehen. Vorliegend wird als Masche jede planmäßige, sich regelmäßig wiederholende Öffnung in der Ebene des textilen Flächengebildes bezeichnet, also auch diejenige Durchgangsöffnung, die in einem gewebten Textil zwischen den Kett- und Schussfäden entsteht. Als Fadenmaterial kann sowohl Naturgarn als auch Kunstfaser zum Einsatz kommen.

Erfindungsgemäß ist das Implantat schlauchförmig ausgebildet, also im Gegensatz zu einem textilen Flächengebilde dreidimensional geformt. Nach einer vorteilhaften Ausgestaltung der Erfindung können nebeneinanderliegende Maschen, die eine Maschenreihe bilden, dabei derart orientiert sein, dass ihre Maschenreihe sich in einem Winkel zur Längserstreckung des schlauchförmigen Implantates bzw. in einem Winkel zu dessen Rotationsachse verläuft. Dadurch erhält das erfindungsgemäße Implantat die vorteilhafte Eigenschaft, dass es sowohl komprimierbar als auch expandierbar ist. Zum Komprimieren wird das schlauchförmige Implantat an seinen axialen Enden in entgegengesetzte Richtungen gezogen, also gestreckt. Dadurch erhält es einen geringeren Durchmesser bei einer größeren Längserstreckung. Zum Expandieren dagegen werden die axialen Enden des Implantats aufeinander zubewegt, das schlauchförmige Implantat also gestaucht. Dadurch gewinnt es einen größeren Durchmesser, wohingegen seine Längserstreckung reduziert wird.

Komprimiert lässt sich das schlauchförmige Implantat zum Beispiel durch einen Trokar minimalinvasiv einführen. Im Körper kann es anschließend hinsichtlich seines Durchmessers mit geringem Aufwand an den Umfang eines zu behandelnden Organs angepasst, seine Form also intraoperativ geändert werden. Damit lässt es sich insbesondere dafür einsetzen, ein Organ stützend zu umschließen. Nach dem Einführen lässt es sich durch Stauchen aufweiten, bis sein Durchmesser ein erforderliches Maß erreicht hat, um das zu stützende Organ zu umgeben. Nach dem Aufschieben und Umhüllen des Organs lässt sich das schlauchförmige Implantat durch Zug komprimieren, dadurch an das zu stützenden Organ anlegen und schließlich fixieren. Das Implantat ist also expandierbar, um sich radial dem Organ anzupassen, und komprimierbar, insbesondere reversibel komprimierbar, um sich zunächst minimalinvasiv in den Körper einbringen und anschießend nach seiner Expansion um das Organ anschmiegen zu lassen.

Seine Expandier- und Komprimierbarkeit bietet eine variable Anpassung des Implantats an organische Strukturen, die sich durch Stauchung und Zug reversibel beeinflussen lässt. Das schlauchförmige Implantat kann vorteilhaft bei langgestreckten, zylindrischen Strukturen eingesetzt werden, beispielsweise bei einem Scheidenstumpfvorfall. Da das schlauchförmige Implantat hohl ist, berührt es nicht zwingend die Stelle des apikalen Scheiden-/Zervixstumpfes, an der sich nach einer Gebärmutterentfernung eine empfindliche Schwachstelle befindet. Beim Auftreffen auf den Zervixstumpf weitet sich das Implantat, bis es sich darüber stülpen lässt. Durch anschießenden Zug umschließt es den Stumpf, passt sich ihm exakt an und ermöglicht so eine tragfähige Aufhängung des Zervixstumpfes durch Fixieren des Implantats zum Beispiel am Os sacrum.

Da die Expandier- und Komprimierbarkeit über die gesamte Länge des schlauchförmigen Implantats vorliegen kann, lässt es sich auch an andere als zylindrisch geformte Strukturen anpassen, zum Beispiel kugelförmige oder unregelmäßige Strukturen. Diese Anpassungsfähigkeit erlaubt den Einsatz des schlauchförmigen Implantats bei einer Vielzahl von zu behandelnden Organen, weil es beispielsweise auch Beutel um Hohlorgane wie das Herz bilden kann. Die offenen Enden des schlauchförmigen Implantats können dabei vorteilhaft als Auslässe für anschließende Gefäße der Hohlorgane dienen.

Das schlauchförmige Implantat lässt sich mit geringem Gewicht und dennoch zugstabil und reißfest ausbilden, so dass es zum Beispiel prolabiertes Körpergewebe dauerhaft unterstützen und in der anatomisch korrekten Lage fixiert halten kann.

Die oben beschriebene Komprimierbarkeit und Expandierbarkeit des schlauchförmigen Implantats rührt aus einer für das erfindungsgemäße Netz typischen Beweglichkeit der maschenbegrenzenden Fäden bzw. Fadenstränge zueinander her. Denn in der Netzebene betrachtet lassen sich die Fadenstränge um ihre Knotenpunkte herum gegeneinander verschwenken, was zu einer Verformung der Maschen führt. Erfindungsgemäß kann das schlauchförmige Implantat ein knotenloses, insbesondere gewirktes, gestricktes, gewebtes oder gelegtes und hier insbesondere ein geklöppeltes oder geflochtenes Netz umfassen. Ein knotenloses Netz bietet den Vorteil, dass die maschenbildenden Fäden bzw. Stränge gegeneinander nicht nur verdrehbar, sondern auch verschiebbar sind, die Maschen also eine veränderbare Größe aufweisen. Darüber hinaus sind auch die Maschen an sich verschiebbar, woraus eine besondere Beweglichkeit und Anpassungsfähigkeit des schlauchförmigen Implantats resultiert. Sie unterstützt die Fähigkeit des an sich schlauchförmigen Implantats, Aufweitungen oder Beutel auszubilden, also entlang seiner Längserstreckung unterschiedliche Durchmesser anzunehmen. Die sowohl rotatorische als auch translatorische Beweglichkeit der Fäden und Fadenstränge relativ zueinander und die Beweglichkeit der Maschen hinsichtlich ihrer Lage, Form und Größe führt dazu, dass sich die Aufweitungen nicht zwingend symmetrisch zur Längsachse des Implantats ausbilden lassen, sondern auch ungleichmäßig und asymmetrisch.

Das erfindungsgemäße Implantat eignet sich vorteilhaft zur medizinischen Anwendung, insbesondere zur Anwendung bei der Vorbeugung und Behandlung eines Organvorfalls im Bereich der Vagina, bevorzugt zur Vorbeugung und Behandlung einer Beckenbodeninsuffizienz, insbesondere eines Level-I-Defekts nach DeLancey.

Entsprechend betrifft eine bevorzugte Verwendung die Anwendung des erfindungsgemäßen Implantats in einem chirurgischen, therapeutischen oder diagnostischen Verfahren. Besonders bevorzugt erfolgt die Anwendung bei einer Zervicosacropexie nach einer suprazervicalen Hysterektomie. Entsprechend betrifft eine weitere Anwendung ein operatives Verfahren zur Durchführung einer Sacrozervico-/-kolpopexie.

Gemäß einer weiteren bevorzugten Ausführungsform eignet sich das erfindungsgemäße Implantat zur Anwendung als zumindest teilweise resorbierbare Organumhüllung, bevorzugt als vollresorbierbare Organumhüllung, z. B. der Leber, der Niere und/oder der Milz.

Nach einer weiteren vorteilhaften Ausgestaltung der Erfindung kann das Implantat eine erste Gruppe von etwa parallel zueinander liegenden Strängen aus mindestens zwei Fäden und eine zweite Gruppe von parallel zueinander liegenden Strängen aus mindestens zwei Fäden umfassen, wobei die zweite Gruppe von Strängen unter Ausbildung von Kreuzungsstellen in einem Winkel zu den Strängen der ersten Gruppe verlaufen, und wobei die Stränge an den Kreuzungsstellen miteinander verflochten sind, indem zumindest ein Faden des einen Stranges zwischen den Fäden des anderen Stranges verläuft. Der Winkel an den Kreuzungsstellen der Stränge ist dabei nicht auf den rechten Winkel festgelegt, sondern kann auch schon im Herstellungszustand nahezu jeden anderen spitzen bzw. stumpfen Winkel annehmen. Die Fäden bzw. Stränge können also trotz Ausbildung von Kreuzungsstellen dort in einem gewissen Rahmen frei gleiten. Dadurch lässt sich - in einer Abwicklung des schlauchförmigen Implantats betrachtet - ein geeignetes textiles Netz herstellen, dessen Maschenform und Maschengröße sich flexibel anpassen lässt. Das dem schlauchförmigen Implantat zugrunde liegende Textil kann sich durch Klöppeln, Wirken, Weben, Legen oder Flechten erzeugen lassen.

Nach einer weiteren vorteilhaften Ausgestaltung der Erfindung können die Stränge jeweils aus miteinander verdrillten Fäden bestehen und in einer Erstreckungsebene des abgewickelten Implantats bzw. orthogonal auf die Mantelfläche des Implantats betrachtet durch Übereinanderliegen jeweils Verdrillungsstellen und dazwischen liegende Schlaufen bilden, wobei ein Faden des einen Stranges zwischen den Fäden einer Schlaufe des anderen Stranges verläuft. Die Fäden eines Stranges, aber auch die Stränge selbst können also innerhalb der Schlaufen des jeweils anderen Strangs frei gleiten. Die Schlaufen definieren dadurch einen Bewegungsraum, der einerseits den Fäden bzw. Strängen einen Spielraum offen hält, den sie andererseits aber auch begrenzen. Ein derart gestaltetes Netz zur Ausbildung des schlauchförmigen Implantats bietet eine ausreichende Stabilität der Maschen untereinander bei gleichzeitig hoher Flexibilität des schlauchförmigen Implantats insgesamt.

Nach einer weiteren vorteilhaften Ausgestaltung der Erfindung kann das Implantat schon herstellungsseitig unterschiedliche Maschenweiten aufweisen. Das dem Implantat zugrunde liegende Netz erhält also eine unterschiedliche bzw. unregelmäßige Maschenverteilung mit Bereichen größerer und Bereichen kleinerer Maschen, die sich sowohl in Längsrichtung also auch in Querrichtung des schlauchförmigen Implantats ergeben können. Mit der unterschiedlichen Maschenweite erhält das schlauchförmige Implantat auch eine unregelmäßige Ausbildung seiner Eigenschaften, insbesondere hinsichtlich seiner Komprimierbarkeit bzw. Expandierbarkeit. Dadurch lassen sich Bereiche der Mantelfläche des Implantats ausbilden, die sich nicht so weit komprimieren oder expandieren lassen wie benachbarte Bereiche. Genauso lassen sich gezielt Abschnitte des Implantats mit kleineren Maschen ausbilden, um einen Matratzeneffekt, also ein Ein- oder Durchdrücken von Netzstrukturen auf dünnwandige Hohlorgane beim Umschließen eines Hohlorgans zu vermeiden.

Nach einer weiteren vorteilhaften Ausgestaltung der Erfindung können die Stränge des Implantats aus monofilen und/oder multifilen und/oder Mehrkomponenten-Fäden ausgebildet sein. Als monofil ist ein Garn zu verstehen, das aus einem einzigen Filament bzw. "einfädig" aufgebaut ist. Als multifil dagegen sind Fäden zu bezeichnen, die aus einem Bündel aus mehreren Einzelfilamenten zusammengesetzt sind. Ein Faden kann also bei gleicher Dicke sowohl monofil als auch multifil ausgebildet sein. Monofile Fäden bieten in der Regel eine gewisse Steifigkeit, was für das optimale Anpassen des schlauchförmigen Implantats im Körper und insbesondere für den Stauchungsvorgang zur Expansion des Implantats von Vorteil ist. Multifile Fäden dagegen sind in der Regel weicher und durch ihren geringeren Fremdkörperreiz für einen Patienten angenehmer "zu tragen". Mehrkomponenten-Fäden schließlich bieten den Vorteil, zwei oder mehrere unterschiedliche Eigenschaften von Fäden, die über diejenigen der Weichheit bzw. der Steifigkeit hinausgehen können, in einem Faden zu vereinen. Diese Eigenschaften können einem Faden bereits bei seiner Herstellung verliehen werden, ohne erst später bei einer Faden- oder Textilbeschichtung und damit über mehrere Herstellungsschritte hinweg kostenintensiv umgesetzt zu werden. Damit lassen sich Implantate herstellen, deren Eigenschaften sich individuell für spezielle Einsatzzwecke anpassen lassen.

Nach einer weiteren vorteilhaften Ausgestaltung der Erfindung können die monofilen oder multifilen oder Mehrkomponenten-Fäden der Stränge zumindest teilweise aus biokompatiblem, resorbierbarem bzw. teilresorbierbarem Material bestehen. Dazu kann beispielsweise eine Hälfte eines Strangs aus einem nicht resorbierbaren Faden, zum Beispiel Polypropylen, und die andere Hälfte aus einem resorbierbaren Material, zum Beispiel Copolymer aus Polyglycolsäure und Poly-ε-Caprolacton bestehen. Durch das resorbierbare Material kann das Implantat teilweise oder vollständig im Körper abbaubar sein. Bei einem teilweisen Abbau des Implantats kann ein leichteres Implantat und damit weniger Fremdmaterial im Köper verbleiben. Alternativ oder zusätzlich kann dadurch ein Implantat geschaffen werden, dessen Eigenschaften sich während seiner Lebensdauer verändern. So können für die Implantation optimale Eigenschaften des Netzes vorgegeben werden, die sich nach der Operation verlieren. Beispielsweise kann sich das Material des Implantats von steif und für einen Operateur gut handhabbar zu weich und körperverträglich verwandeln. In einen resorbierbaren Faden können auch Arzneimittel eingebracht werden, die dann im Körper kontrolliert freigesetzt werden.

Nach einer weiteren vorteilhaften Ausgestaltung der Erfindung können die Stränge bzw. Fäden des Implantats teilweise über eine Färbung verfügen. Sowohl einzelne Fäden also auch einzelne Stränge können ganz oder teilweise gefärbt sein. Stränge können sich dazu zumindest teilweise aus gefärbten Fäden zusammensetzen. Unter Färbung ist dabei eine Gestaltung der Fäden bzw. Stränge zu verstehen, die entweder menschlich oder mithilfe technischer Mittel sichtbar ist bzw. sowohl menschlich als auch mithilfe technischer Mittel. Als technische Visualisierungsmittel steht beispielsweise die MRT-, Röntgenoder Ultraschalltechnologie zur Verfügung. Dadurch lässt sich das Implantat nachträglich insbesondere zu diagnostischen Zwecken sichtbar machen. Dies lässt sich beispielsweise durch Eisenoxidpigmente erreichen, die den Faden im menschlich sichtbaren Bereich kaum einfärben, aber eine postoperative Darstellung ermöglichen. Die Färbung im menschlich sichtbaren Bereich kann der besseren Orientierung im Körper zur Unterscheidung des Implantats von den Organen dienen. Sie kann daher blau, grün, schwarz oder violett sein, weil sich diese Farben von rot, gelb oder weiß im Körper gut abheben.

In Verbindung mit dem oben genannten resorbierbaren bzw. teilresorbierbaren Material kann das schlauchförmige Implantat zunächst eingefärbt und sichtbar gestaltet sein, so dass es sich während eines operativen Eingriffs vom Körpergewebe gut unterscheidet. Später kann es sich durch Resorption bzw. Teilresorption entfärben, um insbesondere unter einer dünnen Haut keinen beispielsweise bläulichen und damit kranken Schimmer zu hinterlassen.

Nach einer weiteren vorteilhaften Ausgestaltung der Erfindung kann das schlauchförmige Implantat über eine Fadenbeschichtung verfügen. Dabei kann es sich um heilungsfördernde Beschichtungen wie zum Beispiel Körperzellen, also eine Blutplasmabeschichtung, oder Phosphorylcholin oder körpereigenen Verbindungen handeln. Alternativ können hydrophobierende Beschichtungen wie zum Beispiel Polycaprolacton-Polyglycolsäure vorgesehen sein. Weiter alternativ können Metall-, Metalloxid-, Keramik- oder Carbon-Beschichtungen wie z. B. Titandioxidbeschichtungen aufgebracht sein, die entweder die Oberfläche verträglicher machen oder aber eine andere, beispielsweise antibakterielle Wirkung auf das Implantat haben wie die Hemmung von Bakterienbesiedlung des Implantats bei Silber- oder Kupferbeschichtungen. Eine antimikrobielle bzw. antibakterielle Beschichtung wie z. B. Chlorhexidin oder Polyhexanid kann ebenfalls die Besiedelung des Implantats mit Bakterien bzw. die Ausbreitung einer Infektion verhindern. Schließlich können antiadhäsive Bestandteile wie zum Beispiel PTFE, Collagen oder resorbierbare Beschichtungen ein Anwachsen am Darm oder sonstige Adhäsionen verhindern.

Nach einer weiteren vorteilhaften Ausgestaltung der Erfindung kann das Implantat über daran angebrachtes chirurgisches Nahtmaterial oder benadeltes Nahtmaterial oder eine daran angebrachte Nadel als Mittel bzw. Hilfsmittel zur Fixierung des Implantats an einem Organ verfügen. Mit einer derartigen Ausrüstung lässt sich das Implantat nach Positionierung auf dem zu stützenden Organ im Körper festlegen, ohne dass dafür das weitere minimalinvasive Einbringen von Nahtmaterial und/oder einer Nadel erforderlich wäre.

Eine wesentliche Eigenschaft des erfindungsgemäßen schlauchförmigen Implantats ist seine reversible Verformbarkeit, durch die es komprimierbar und expandierbar ist. Dazu lässt sich das dem schlauchförmigen Implantat zugrunde liegende Netzmaterial knotenlos ausbilden. Nach einer dazu alternativen oder weiteren Ausgestaltungsform der Erfindung kann das erfindungsgemäße Implantat elastisches Fadenmaterial zur Ausbildung der Textil- bzw. Netzfläche umfassen. Grundsätzlich lassen sich damit auch geknotete Netze zur Ausbildung des schlauchförmigen Implantats einsetzen, die ihre Verformbarkeit über das elastische Fadenmaterial erhalten. Aber auch bei einem knotenlosen Netzmaterial kann das elastische Fadenmaterial für eine weitere Weichheit bzw. Verformbarkeit des schlauchförmigen Implantats vorteilhaft eingesetzt werden.

Die erfindungsgemäße Netzstruktur und die Ausbildung der Stränge und Fäden des schlauchförmigen Implantats führt zu seiner vorteilhaften reversiblen Anpassungsfähigkeit hinsichtlich seines Durchmessers, die einerseits geeignet große Maschen in einem stützenden Abschnitt des Implantats hinterlässt und andererseits zur Ausbildung eines zugstabilen Strangs in einem anschließenden Abschnitt führt. Die Maschengröße bzw. -weite ist wichtig für die Einheilung, der zugstabile Strang lässt erst eine Aufhängung des zu stützenden Organs zu. Die Maschenweite sollten zwischen 0,8 mm bis 8 mm liegen, denn je nach chemischem Grundmaterial für den Faden erkennt der Körper Abstände kleiner 0,8 mm nicht mehr als Einzelfäden, sondern schirmt den Faden als Fremdkörper durch einen "Kompletteinbau" mit einer Narbenplatte ab. Fadenabstände innerhalb einer Masche von mehr als 8 mm sorgen für ein sehr loses Netz und einen sogenannten Matratzeneffekt, bei dem sich die großen Maschen durch dünne Organhaut drücken und so als Muster am Organ abzeichnen können. Zudem ermöglicht die Erfindung eine vorteilhafte Eigenschaftsmischung für ein textiles Implantat, weil selbst innerhalb einer Materialart ein leichtgewichtiges, dennoch monofiles und gleichzeitig grobmaschiges Netz ausgebildet werden kann, das eine für ein Implantat wünschenswerte Rigidität aber auch eine Formvariabilität hat.

Durch die Verwendung von sehr dünnen Einzelfäden kann das Implantat eine patientenverträgliche Weichheit erhalten. Sinnvolle Fadenstärken reichen von 0,07 mm bis 1 mm, wobei auch durchaus in den Randbereichen sinnvolle Varianten denkbar sind. Denn je dicker die Fäden, desto besser funktioniert die reversible Anpassung des Implantats, desto eher aber wird es als steifer Fremdkörper angesehen. Umgekehrt sind dünnere Fäden für diese Anwendung körperverträglicher, aber als Implantat schwerer zu handhaben.

Eine Lösung dieses Zielkonflikts bietet die Teilresorption bzw. Kombination von dünnen dauerhaften Fäden und resorbierbaren dicken Fäden, wie z. B. teilresorbierbare Bikomponentenfäden oder die Verarbeitung von zwei Fäden nebeneinander, wovon einer resorbierbar und der andere nicht resorbierbar ist. Damit lassen durch die beschriebenen Ausführungsformen technologische Idealanforderungen an ein Implantat erfüllen.

Nach einer weiteren vorteilhaften Ausgestaltung der Erfindung kann das schlauchförmige Implantat über ein Formgedächtnis verfügen, das das Implantat in eine bestimmte Form "vorspannt" oder vorformt. Das Implantat kann also herstellungsseitig bereits in eine bevorzugte Form gebracht werden, die sich unter Zug oder Druck in der oben beschriebenen Weise grundsätzlich beeinflussen und verändern lässt, in die das Implantat aber ohne Krafteinwirkung von außen, sei es durch einen Operateur oder durch eine ummantelte Körperstruktur, wieder zurückkehrt. Grundsätzlich sind unterschiedliche und in Längsrichtung des schlauchförmigen Implantats betrachtet auch unregelmäßige Formen für seine Vorspannung möglich, insbesondere die Komprimierungsrichtung oder die Expansionsrichtung. Unter den letzten beiden Möglichkeiten ist die Komprimierungsrichtung zu bevorzugen, weil das Implantat zum einen in der komprimierten Form bequem minimal invasiv eingesetzt werden kann, sich zum zweiten in einem Stützabschnitt selbsttätig an die zu stützende Körperstruktur anlegt und zum dritten in einem Befestigungsabschnitt einen schmalen Strang zur Befestigung der zu stützenden Körperstruktur beispielweise an einem Knochen ausbildet. Damit kann die Handhabung des erfindungsgemäßen Implantats unterstützt und erleichtert werden.

Das Prinzip der Erfindung wird im Folgenden anhand einer Zeichnung beispielshalber noch näher erläutert. In der Zeichnung zeigen:
- Figuren 1a bis 1e:: anatomische Verhältnisse im Zusammenhang mit einer Gebärmutterentfernung und mit dem Einsatz eines erfindungsgemäßen Implantats,
- Figuren 2a bis 2b:: Ausschnitte aus einem erfindungsgemäßen Implantat,
- Figur 3:: die Struktur des schlauchartigen Implantats,
- Figuren 4 bis 6:: vereinfachte Darstellungen eines Ausschnitts des Implantats gemäß Figur 2,
- Figuren 7, 8:: Querschnittsformen desselben Implantats, und
- Figuren 9 bis 12:: einen Implantationsvorgang des erfindungsgemäßen Implantats.

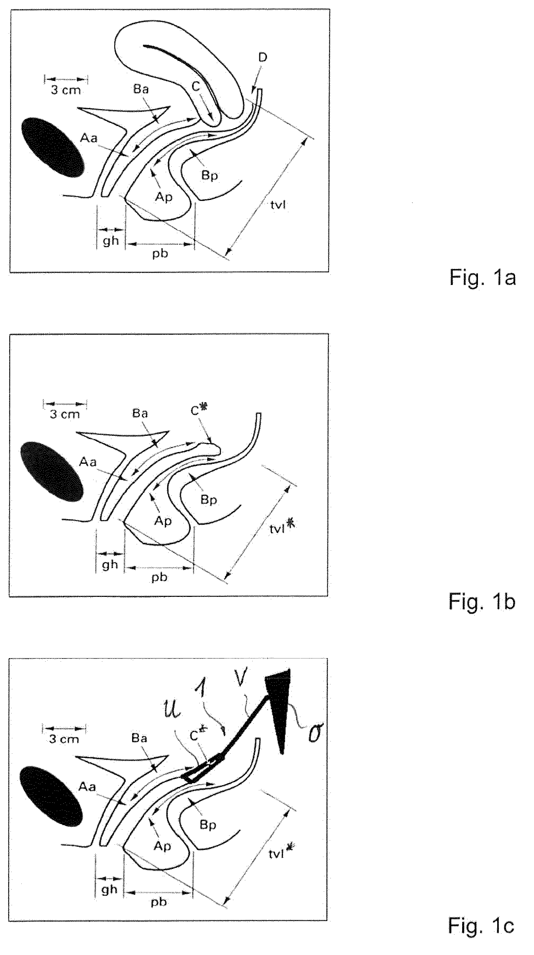

Figur 1a stellt stark schematisiert die Lage einer Gebärmutter in einem unbehandelten Zustand dar. Sie entstammt der Darstellung des "Pelvic Organ Prolapse Quantification System" (POP-Q) in der Fachveröffentlichung "Journal of Medicine and Life", Vol. 4, No.1, January-March 2011, pp.75-81. Figur 1a zeigt eine natürliche Scheidenlänge tvI, die bis zu einem Gebärmutterhals C reicht.

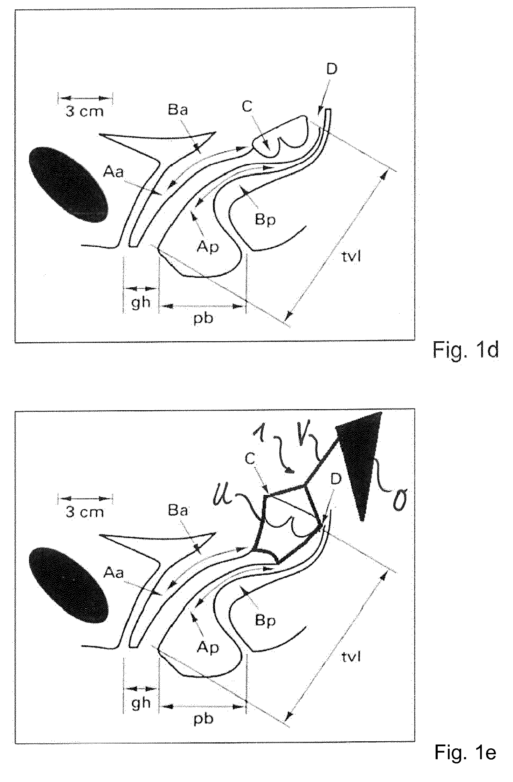

Figur 1 b gibt die Situation nach einem operativen Eingriff wieder, bei dem die Gebärmutter vollständig entfernt wurde (totale Hysterektomie). Daraus resultiert eine verkürzte Scheidenlänge tvI*, die sich bis zu einem Scheidenstumpf C* erstreckt. Hier empfiehlt sich der Einsatz des erfindungsgemäßen chirurgischen Implantats 1, um einen Scheidenstumpfvorfall zu vermeiden. Dazu wird gemäß Figur 1c das schlauchförmige Implantat 1 mit einem vorderen Abschnitt U auf den Scheidenstumpf C* aufgeschoben und mit einem hinteren Abschnitt V am Kreuzbein bzw. Os sacrum O befestigt.

Durch suprazervicale Hysterektomie, also eine Gebärmutterentfernung unter Erhalt des Gebärmutterhalses C, kann gemäß Figur 1c die natürliche Scheidenlänge tvI aufrechterhalten werden. Dazu ein erfindungsgemäßes schlauchförmiges Implantat 1 mit einem vorderen Abschnitt U über den Gebärmutterhals C gestülpt und mit einem hinteren Abschnitt V am Kreuzbein bzw. Os sacrum O fixiert.

Operationstechniken, den Scheidenstumpf C* am Os sacrum aufzuhängen und somit einem Scheidenstumpfvorfall zu stabilisieren oder diesem vorzubeugen (Sacrokolpopexie), haben einen festen Platz in den Leitlinien der Deutschen Gesellschaft für Gynäkologie und Geburtshilfe gefunden. Traditionell wird dazu ein offener Abdominalschnitt geführt. Alternativ kann der Eingriff inzwischen auch minimalinvasiv in laparoskopischer Technik über Trokarsysteme durchgeführt werden. Dies gilt umso mehr, als man in Zeiten von Warnmeldungen über vaginal implantierte Prolaps-Netze durch die amerikanische Gesundheitsbehörde FDA wieder verstärkt diese Operationsmethode der Sacrokolpopexie beschreitet.

Operateure verwendeten hierzu zunächst zugeschnittene rechteckige Herniennetze, die in Form eines Schnabels gebracht wurden, zum Beispiel den sog. Kieler Entenschnabel mit zwei schnabelartig aufklappbaren Fortsätzen und einem dritten Fortsatz zur Fixierung am Os sacrum. Später wurden spezielle Y-förmige Netze gefertigt, die ebenfalls einen schnabelartigen Teil zur Fixierung des Scheiden- oder des Zervixstumpfes und einen langen Teil zur Fixierung am Os sacrum aufwiesen: Die kurzen Enden des Y umschließen die Scheide auf der Ober- und Unterseite und der lange Teil des Y wird sacral fixiert.

Dem Operateur bieten sich allerdings unterschiedliche Probleme, die während der Anwendung solcher Implantate auftreten. Zugeschnittene Herniennetze lassen eine individuelle Anpassung nur sehr begrenzt zu. Zudem können die Implantate aufgrund ihrer Instabilität nicht minimalinvasiv verwendet werden. Manche fertig konfektionierte Y-Netze hingegen lassen sich durch einen Trokar einführen. Dann aber lässt sich eine intraoperative Anpassung der Implantate in den beengten Platzverhältnissen und eingeschränkt durch die Freiheitsgrade der Instrumentenführung bei laparoskopischer Technik nur schwer vornehmen.

Konstruktionsbedingt sitzt die Mehrzahl der Y-Netze am Scheiden- oder Zervixstumpf genau an der Stelle direkt auf, an der die Gebärmutter entfernt wurde. Ist die Gebärmutterentfernung in einem vorangegangenen Eingriff mit ausreichendem zeitlichem Abstand erfolgt, gibt es nur ein geringes Komplikationsrisiko. Wird die Gebärmutterentfernung und die Sacrokolpopexie allerdings in einer Operationssitzung durchgeführt, besteht an dieser Stelle die Gefahr einer Erosion. Dabei arbeitet sich das Netzgewebe durch die Wunde am Scheidenstumpf durch. Oder aber es drohen Fisteln, also Gänge von der offenen Scheide zu anderen Hohlorganen wie Blase oder Darm.

Bei allen Y-Netzen ist eine Fixierung der Fortsätze auf der Scheide mit mehreren Nähten an der Scheidenvorder- und -hinterwand oder anderen Fixierungshilfen wie z. B. Tackern notwendig. Dies erfordert sehr viel Geschick des Operateurs und bringt eine Belastung der oft atrophen Scheidenhaut mit sich.

Ideal ist demnach ein Implantat, das sich minimalinvasiv einführen und dann intracorporal ohne weitere Hilfsinstrumente, individuell anpassen und am Scheiden-/Zervixstumpf fixieren lässt. Die individuelle Anpassung muss reversibel erfolgen können, bis der anatomisch optimale Zustand erreicht ist. Die Maschengröße muss dabei so groß sein, dass sich eine flexible, elastische Schicht aus Körpergewebe und Implantat bildet. Das Implantat muss zugstabil sein und somit das prolabierte Organ wieder dauerhaft in seiner ursprünglichen, anatomisch korrekten Lage fixieren. Das sacral zu fixierende Ende muss deshalb stabil und reißfest sein.

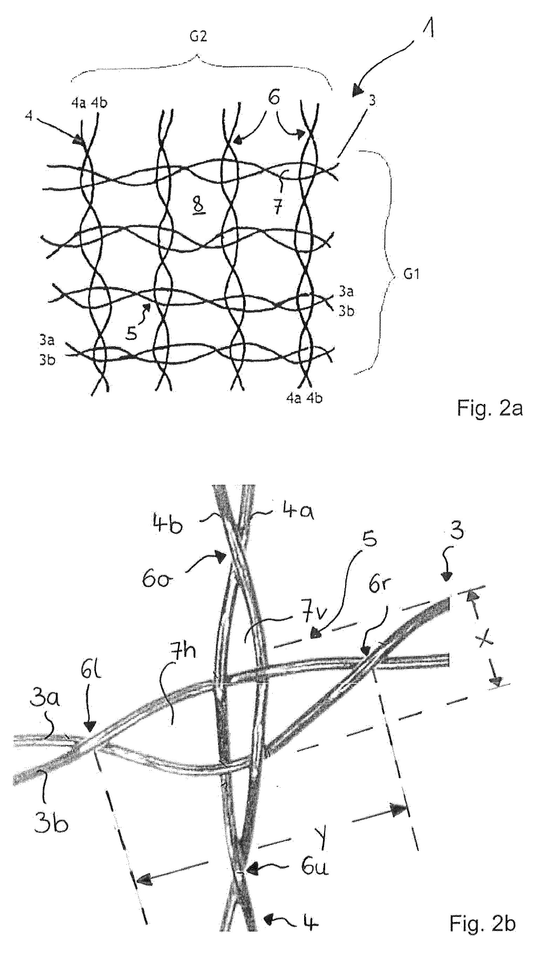

Diesen Anforderungen wird das erfindungsgemäße schlauchförmige Implantat gerecht: Fig. 2a zeigt einen Ausschnitt aus einem erfindungsgemäßen Implantat 1, das durch Klöppeln hergestellt wurde, in einer zur Erstreckungsebene des Ausschnitts orthogonalen Ansicht bzw. in einer lotrechten Richtung auf die Mantelfläche des schlauchförmigen Implantats 1 betrachtet. Sie dient der Verdeutlichung der durch das Klöppeln erzeugten Bindungsart. Das Implantat besteht demnach aus einer Vielzahl von jeweils nebeneinander angeordneten Gruppen G1 und G2 von sich kreuzenden Strängen 3 und 4, wobei die Stränge 3 der Gruppe G1 horizontal und die Stränge 4 der Gruppe G2 vertikal verlaufen. Sie stehen damit orthogonal aufeinander und kreuzen sich an Kreuzungsstellen 5 in einem weitgehend rechten Winkel. Zwei benachbarte Stränge 3 kreuzen sich mit zwei benachbarten Strängen 4 an vier Kreuzungsstellen 5 und schließen zwischen sich eine Masche 8 ein.

Die Stränge 3, 4 bestehen jeweils aus zwei Fäden 3a und 3b bzw. 4a und 4b. Die Fäden 3a, 3b bzw. 4a, 4b eines Strangs 3, 4 sind jeweils miteinander verdrillt, so dass sie in der Ansicht der Figur 2a an Verdrillungsstellen 6 abschnittsweise übereinander liegen und sich zwischen den Verdrillungsstellen 6 in beiden Strängen 3, 4 Schlaufen 7 ergeben.

Figur 2b zeigt eine einzelne Kreuzungsstelle 5 zur Verdeutlichung. Erfindungsgemäß kreuzen sich dort jeweils zwei Schlaufen 7 unterschiedlicher Stränge 3, 4. Eine vertikal verlaufende Schlaufe 7v bildet sich zwischen einer oberen Verdrillungsstelle 6o und einer unteren Verdrillungsstelle 6u, eine horizontal verlaufende Schlaufe 7h liegt zwischen einer linken Verdrillungsstelle 6l und einer rechten Verdrillungsstelle 6r. Die Stränge 3, 4 sind derart miteinander verbunden, dass der Faden 4a, der an der oberen Verdrillungsstelle 6o des Stranges 4 unter dem Faden 4b verläuft, durch die Schlaufe 7 des Stranges 3 und zwischen den Fäden 3a, 3b hindurch, und zwar über den Faden 3b und unter dem Faden 3a hindurch verläuft, um an der unteren Verdrillungsstelle 6u auf dem Faden 4b zu liegen. In sinngemäß umgekehrter Weise verläuft der Faden 4b. Die Kreuzungsstellen 5 bilden damit quasi Gelenkpunkte zwischen dem Strang 3 und dem Strang 4.

Die Fäden 3a, 3b des Strangs 3 verlaufen in prinzipiell vergleichbarer Weise: Der Faden 3a verläuft relativ zum Faden 3b und zu den Fäden 4a, 4b sinngemäß wie der Faden 4a, jedoch nicht von oben nach unten wie jener, sondern von links und ausgehend von der linken Verdrillungsstelle 6l nach rechts zur rechten Verdrillungsstelle 6r. In sinngemäß umgekehrter Weise verläuft der Faden 3b.

Die Fäden 3a, 3b bzw. 4a, 4b verlaufen also sowohl abwechselnd oben und unten und changieren von einer dem Betrachter zugewandten Oberseite zu einer ihm abgewandten Unterseite als auch abwechselnd links und rechts. Zur Ausbildung der Schlaufe 7v befindet sich z. B. der Faden 4b vor der oberen Verdrillungsstelle 6o links, bildet in der Schlaufe 7v deren rechten Rand, kreuzt den Faden 4a an der Verdrillungsstelle 6u erneut und befindet sich danach wieder links. Entsprechendes gilt für die Fäden 3a, 3b des Strangs 3.

Auf diese Weise sind die Schlaufen 7 eines Strangs 3, 4 zwischen den Verdrillungsstellen 6 sowohl desselben Strangs 3, 4 als auch zwischen denjenigen des anderen Strangs 4, 3 eingeschlossen. Damit durchdringen sich die Schlaufen 7 unterschiedlicher Stränge 3, 4 an den Kreuzungsstellen 5 gegenseitig. Die Fäden 3a, 3b liegen in einem unbelasteten Ausgangszustand zur Ausbildung der Schlaufen 7 nicht unmittelbar aneinander an, sondern bauchen zwischen den Verdrillungsstellen 6l, 6r bis auf einen maximalen Abstand X zueinander aus. Die Verdrillungsstellen 6l, 6r liegen auf dem Strang 3 in einem Abstand Y voneinander entfernt, der etwa dem dreifachen des Abstands X entspricht. Dadurch ist die Schlaufe 7h des Strangs 3 innerhalb der Schlaufe 7v des Strangs 4 geringfügig verschiebbar und umgekehrt.

Durch ein Stauchen des Implantats 1 in Richtung der Stränge 3 oder 4 vergrößern sich die Abstände X der Fäden 3a, 3b; 4a, 4b, wodurch sich die Schlaufen 7 weiten und sich der Abstand Y verkürzt. Bei einer entsprechenden Zugbelastung dagegen verringern sich die Abstände X, bis die Fäden 3a, 3b, 4a, 4b weitgehend aneinander anliegen, die Schlaufen 7 also nahezu geschlossen sind. Gleichzeitig vergrößern sich die Abstände Y. Das gegenseitige Durchdringen der Schlaufen 7 führt zu einer hohen Reibung zwischen den Fäden 3a, 3b, 4a, 4b an jeder Verdrillungsstelle 6 und an jeder Schlaufe 7, die die Zugbelastung im Implantat 1 gleichmäßig verteilt. Dadurch stellt das Implantat 1 ein zugstabiles Implantat dar, das die Zugkräfte dauerhaft abfangen und somit eine ausreichende Fixierung und Unterstützung von Körperstrukturen übernehmen kann.

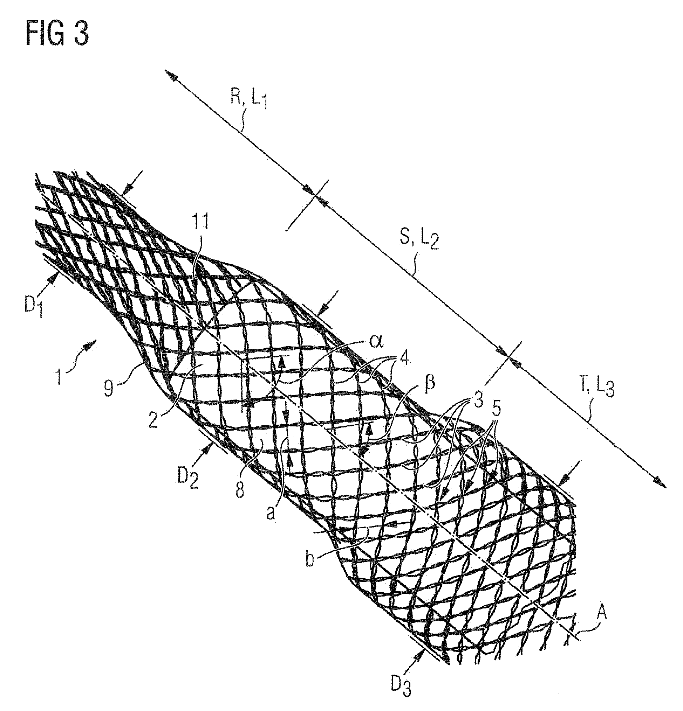

Figur 3 veranschaulicht die Struktur des schlauchartigen Implantats 1 aus einem Klöppelnetz als Hülle 9 mit einem eingeführten weißen Rohr 2 in drei unterschiedlich belasteten Abschnitten R, S und T. Erfindungsgemäß ist die Hülle 9 des Implantats 1 schlauchartig um eine Längsachse A ausgebildet, wobei sowohl die Stränge 3 als auch die Stränge 4 in einem Winkel β zur Längsachse A verlaufen. In dem mittleren Abschnitt S des Implantats 1, in dem das Implantat 1 unbelastet ist, liegt der Winkel β bei etwa 45°, der Winkel α, also derjenige der Stränge 3, 4 zueinander, bei etwa 90°. Das schlauchartige Implantat 1 weist einen Durchmesser D2 und weitgehend quadratische Maschen 8 mit einem Abstand a benachbarter Kreuzungspunkte 5 auf einem Strang 4 und mit einem Abstand b benachbarter Kreuzungspunkte 5 auf einem Strang 3 auf.

Es zeigt sich hier, dass das schlauchförmige Implantat 1 im Abschnitt L2 mit seiner textilen Hülle 9 mit dem das Körpergewebe darstellenden Rohr 2 in Kontakt ist. Am Ende des Rohrs 2 bzw. an seiner nicht erkennbaren stirnseitigen Öffnung im Abschnitt L1 dagegen bildet sich durch die dortige Verjüngung des Implantats 1 ein weitgehend kegelförmiger Freiraum 11 innerhalb der Hülle 9, der stets freigehalten wird. In ihm kommt beispielsweise die Wunde am Scheidenapex bei einer Sacrokolpopexie zu liegen. Sie bleibt dadurch von der Hülle 9 unberührt und damit insbesondere mechanisch unbelastet.

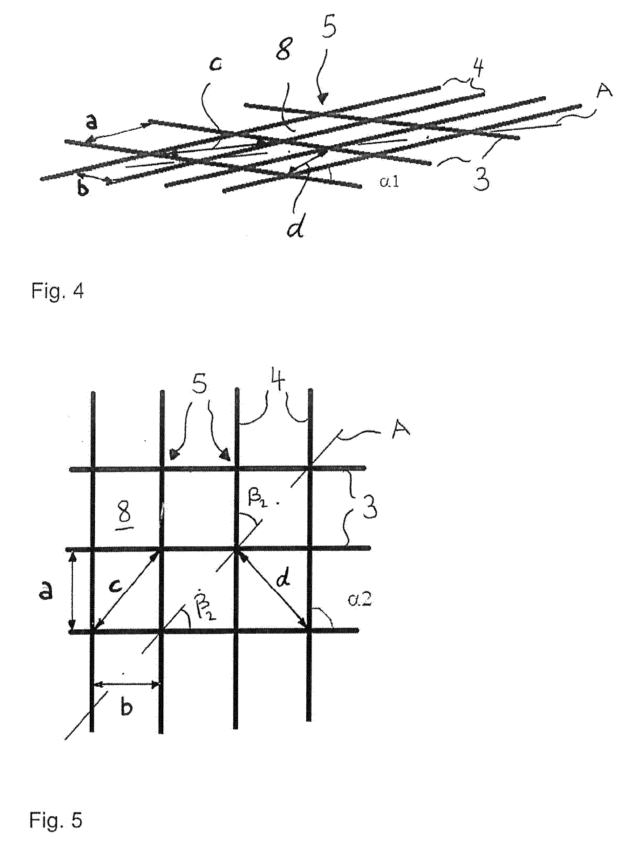

Figur 5 zeigt den Abschnitt S in einer vereinfachten Darstellung, in der drei horizontal verlaufende Stränge 3 und vier vertikal verlaufende Stränge 4 jeweils als Linien symbolisiert sind. Außerdem wird davon ausgegangen, dass sie an ihren Kreuzungsstellen 5 gegeneinander weitgehend unverschiebbar gehalten sind, die Abstände a und b nebeneinanderliegender Kreuzungspunkte 5 also grundsätzlich nahezu konstant bleiben. Die Stränge 3, 4 schließen demnach einen rechten Winkel α₂ ein, der dem Abschnitt S eine mittlere Länge L2 und einen mittleren Durchmesser D2 (vgl. Figur 3) verleiht. Gegenüber der Längsachse A des Implantats 1 verlaufen die Stränge 3, 4 jeweils in einem Winkel β₂ von etwa 45°. Auch die Abstände zwischen zwei sich innerhalb einer Masche 8 diagonal gegenüberliegenden Kreuzungspunkten 5, nämlich der Abstand c in Achsenrichtung und der rechtwinklig dazu bestehende Abstand d - er verläuft in Durchmesserrichtung -, sind gleich.

Ein Strecken bzw. eine Zugbelastung des schlauchartigen Implantats 1 - in dem oberen Abschnitt R der Figur 3 dargestellt - führt an den Kreuzungspunkten 5 zu einer Veränderung sowohl des Winkels α als auch des Winkels β und der Abstände c, d: Die Winkel α, die in einer Masche 8 in Richtung der Längsachse A einander gegenüberliegend, verkleinern sich, die beiden anderen Winkel derselben Masche 8 dagegen werden aufgeweitet, weil die Stränge 3, 4 im spitzeren Winkel β₂ gegenüber der Längsachse A verlaufen. Folglich vergrößert sich der Abstand c zweier in Achsenrichtung einander gegenüberliegender Kreuzungspunkte 5, wohingegen sich der Abstand d verringert. Das schlauchartige Implantat 1 erhält dadurch lange, in Achsrichtung A gestreckte dünne Maschen 8 und einen kleineren Durchmesser D1, was insgesamt zu einer Verlängerung des Implantats 1 führt. (vgl. auch Figur 4)

Figur 4 zeigt den Abschnitt R in jener vereinfachten Darstellung, in der Linien die Stränge 3, 4 symbolisieren. Sie zeigt einen spitzen Winkel α₁, der im Abschnitt R eine große Länge L1 und einen kleinen Durchmesser D1 (Figur 3) nach sich zieht, wobei die Abstände a und b zwischen zwei Knotenpunkten 5 desselben Strangs 3, 4 als konstant angenommen werden. Das Ergebnis ist ein langes, dünnes Implantat 1, das gut minimalinvasiv in eine Trokarhülse eingeführt werden kann.

Bei einem Stauchen des schlauchartigen Implantats 1 in dem unteren Abschnitt T der Figur 3 dagegen vergrößern sich die Winkel α, die in einer Masche 8 in Richtung der Längsachse A einander gegenüberliegend, die beiden anderen Winkel α derselben Masche 8 dagegen nehmen ab, weil die Stränge 3, 4 nun in einem flacheren Winkel β gegenüber der Längsachse A verlaufen. Bei wiederum konstanten Abständen a, b wird nun der Abstand c zweier in Achsenrichtung einander gegenüberliegender Kreuzungspunkte 5 reduziert, während der Abstand d wächst. Das schlauchartige Implantat 1 erhält dadurch kurze, in Achsrichtung A gestauchte "dicke" Maschen 8 und einen größeren Durchmesser D3.

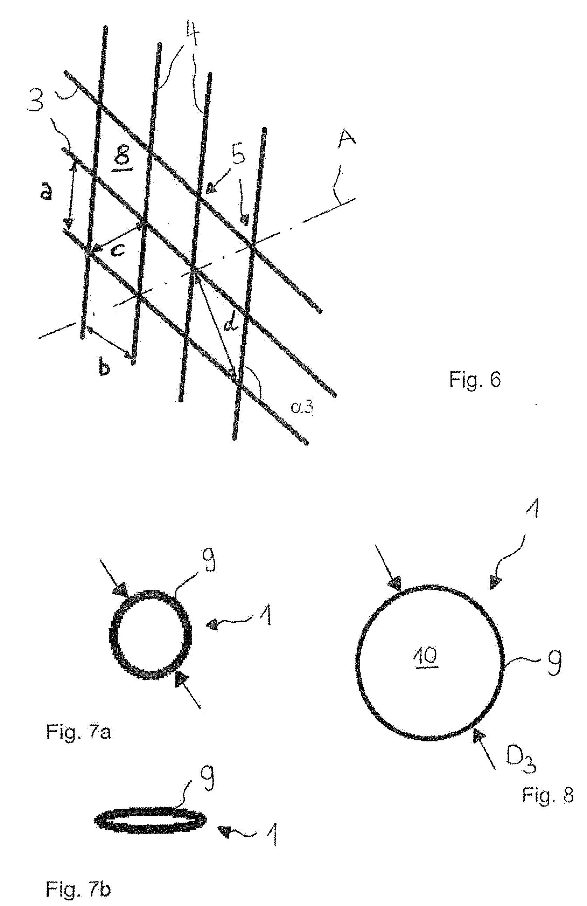

In der wiederum vereinfachten Darstellung des Abschnitts T gemäß Figur 6 zeigt sich ein stumpfen Winkel α₃, der beim Abschnitt T eine geringe Länge L3 und einen großen Durchmesser D3 (s. Figur 3) nach sich zieht, wobei die Abstände a und b wieder als unveränderbar angenommen sind. D3 und L3 werden in der Praxis dadurch erreicht, dass man das schlauchartige Implantat 1 gegen ein Hindernis schiebt.

Klöppeln als Bindungsart führt also bei dem schlauchartigen Implantat dazu, dass sich die Stränge 3, 4 in Achsrichtung A betrachtet in einem Bereich zwischen langen, dünnen Maschen 8 (Durchmesser D1) und kurzen, dicken Maschen 8 (Durchmesser D3) verformen lassen. Dazu verschwenken sich die Stränge 3 bzw. Fadensysteme G1 (s. Figur 2a) einerseits und die Stränge 4 bzw. Fadensysteme G2 andererseits in den Kreuzungspunkten 5 als Gelenkpunkten gegeneinander. Weil die Abstände a, b innerhalb der Fadensysteme G1 (Abstand a) bzw. G2 (Abstand b) nahezu konstant sind, ändern sich mit den Winkeln α, β, den diagonalen Abständen c, d und den Durchmessern D auch die Gesamtlängen L.

Tatsächlich weisen die Fäden 3a, 3b, 4a, 4b an den Kreuzungsstellen 5 durch Veränderung der Abstände X, Y (vgl. Figur 2b) ebenfalls eine geringfügige Bewegungsmöglichkeit auf. Dadurch verändern sich tatsächlich auch die Abstände a, b (vgl. Figuren 4 bis 6) etwas. Aufgrund von Reibung zwischen den miteinander verflochtenen Fäden 3a, 3b, 4a, 4b an den Kreuzungsstellen 5 tritt diese Änderung allerdings überwiegend erst unter größeren Kompressions- oder Expansionskräften auf, also jeweils kurz vor Erreichen der Durchmesser D1 oder D2. Ist auch die Bewegungsmöglichkeit der Fäden 3a, 3b, 4a, 4b an den Kreuzungsstellen 5 erschöpft, erweist sich das Implantat 1 insbesondere unter Zugbelastung, also wenn schon der minimale Durchmesser D1 erreicht ist, als quasi unelastisch bzw. unnachgiebig.

Figur 7a zeigt eine Schnittansicht des schlauchartigen Implantats 1 mit dem Minimaldurchmesser D1 im Abschnitt R der Figur 2. Durch seitlichen bzw. radialen Druck auf eine Hülle 9 des Implantats 1 oder bei seiner Fixierung lässt es sich ohne Kraftanstrengung zu einem flachen Schlauch mit einem elliptischen Querschnitt gemäß Figur 7b formen.

Figur 8 stellt eine Schnittansicht des Implantats 1 mit dem Maximaldurchmesser D3 im Abschnitt T dar. Davon ausgehend kann sich das Implantat 1 einer zu fixierenden Körperstruktur anpassen.

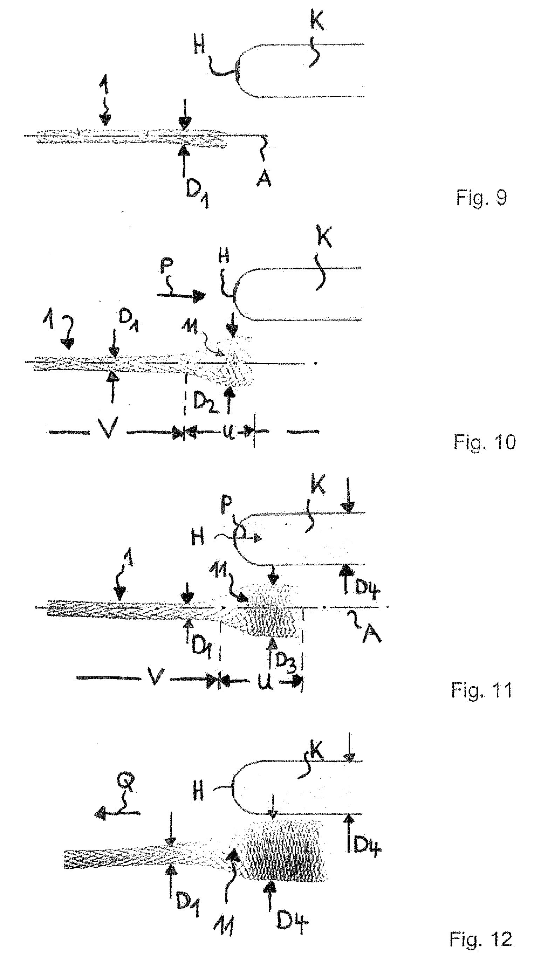

Die Figuren 9 bis 12 zeigen die Funktionsweise des schlauchartigen Implantats 1 während eines minimalinvasiven Implantationsvorganges mithilfe eines nicht dargestellten Trokars. Figur 9 symbolisiert ein zu stützendes Körperteil K, zum Beispiel einen Vaginalstumpf. Nach einer Hysterektomie befindet sich an dessen Spitze eine empfindliche Hysterektomienaht H.

Während einer Durchtrittsphase durch eine Trokarhülse weist das Implantat 1 zunächst den geringsten Durchmesser D1 auf, wobei D1 kleiner ist als der Innendurchmesser des Trokars, beispielsweise 5 bis 10 mm. Mit dem Durchmesser D1 tritt das Implantat 1 aus der Trokarhülse aus und gelangt in eine Bauchhöhle.

Beim Auftreffen des schlauchartigen Implantats 1 in Pfeilrichtung P auf ein Hindernis, zum Beispiel auf eine Bauchwand oder auf das zu stützende Körperteil K, wird es gemäß Figur 10 gestaucht. Gemäß der obigen detaillierten Beschreibung vergrößert sich dabei der Durchmesser D1 eines vorderen Abschnitts U des Implantats 1 zunächst auf den Durchmesser D2, während der hintere Abschnitt V seinen geringen Durchmesser D1 behält.

Dabei bildet sich bereits der kegelförmige Freiraum 11 aus (vgl. Figur 3). In ihn kann beispielweise die bei einer Sacrokolpopexie besonders empfindliche Hysterektomienaht H berührungslos und damit komplikationsarm eintauchen. Auch bei der weiteren Manipulation des Implantats 1 bleibt der Freiraum 11 aufrechterhalten.

Figur 11 bildet den Vorgang der Adaptation zwischen dem Implantat 1 und dem zu stützenden Körperteil K ab. Liegt das Implantat 1 bestimmungsgemäß am Körperteil K an, lässt sich der vordere Abschnitt U des Implantats 1 durch weiteres Vor- bzw. Aufschieben in Pfeilrichtung P auf den Durchmesser D3 aufweiten und um das Körperteil K mit seinem demgegenüber geringeren Durchmesser D4 stülpen. Die Länge des Abschnitts U in Achsrichtung A hängt davon ab, welche anatomischen Verhältnisse oder chirurgischen Vorgaben vorliegen, wird also bestimmt durch die Länge des Körperteils K, zum Beispiel durch die Länge eines Scheidenstumpfs oder durch eine nur kappenartige Fixierung einer Zervix.

Nach dem korrekten Positionieren des Implantats 1 bzw. seines Abschnitts U wird es gemäß Figur 12 an seinem Abschnitt V in Pfeilrichtung Q zurückgezogen. Dabei zieht sich der Abschnitt U zusammen, passt also seinen ursprünglichen Durchmesser D3 automatisch an den geringeren Durchmesser D4 des zu unterstützenden Körperteils K an und umschließt es eng. Die Größe der Maschen des Implantats 1 verhindert eine Einschnürung und sorgt für eine weitestgehend gleichmäßige Druckverteilung und damit zu einem sicheren Halt der zu unterstützenden Körperstruktur K. Der verbleibende Abschnitt V ist so gefertigt, dass er mit separaten Fäden oder durch Tackern an tragenden Körperstrukturen, zum Beispiel am Os sacrum (vgl. Figur 1c) pexiert werden kann und somit das zu stützende Körperteil K, das vom Abschnitt U gehalten wird, dauerhaft stabilisiert, aufhängt und/oder unterstützt.

Da es sich bei dem vorhergehenden, detailliert beschriebenen Implantat um ein Ausführungsbeispiel handelt, kann es in üblicher Weise vom Fachmann in einem weiten Umfang modifiziert werden, ohne den Bereich der Erfindung zu verlassen. Insbesondere können auch die konkreten Ausgestaltungen der Knoten bzw. Maschenbindungen in anderer Form als in der hier beschriebenen erfolgen. Ebenso kann das Implantat in einer anderen Dimensionierung ausgestaltet werden, wenn es auf andere Organe angewendet oder es aus zum Beispiel Handhabungsgründen notwendig ist. Weiter schließt die Verwendung der unbestimmten Artikel "ein" bzw. "eine" nicht aus, dass die betreffenden Merkmale auch mehrmals bzw. mehrfach vorhanden sein können.

### Bezugszeichenliste

- 1: Implantat
- 2: Rohr
- 3: Strang
- 3a, 3b: Faden des Strangs 3
- 4: Strang
- 4a, 4b: Faden des Strangs 4
- 5: Kreuzungsstelle
- 6: Verdrillungsstelle
- 7: Schlaufe
- 8: Masche
- 9: Hülle
- 10: Innenraum
- 11: Freiraum

- A: Längsachse
- C: Gebärmutterhals
- C*: Scheidenstumpf
- D1, D2...: Durchmesser des Implantats
- G1: Gruppe von Strängen 3
- G2: Gruppe von Strängen 4
- H: Hysterektomienaht
- K: zu stützendes Körperteil
- L1: Länge des Implantats bzw. des Abschnitts R
- L2: Länge des Implantats bzw. des Abschnitts S
- L3: Länge des Implantats bzw. des Abschnitts T
- O: Kreuzbein bzw. Os sacrum
- R, S, T, U, V: Abschnitte des Implantats 1
- X: Abstand
- Y: Abstand
- α, α1...: Winkel zwischen den Strängen 3, 4 an den Kreuzungsstellen 5
- β: Winkel der Stränge 3, 4 gegenüber der Längsachse A
- a, b, c, d: Abstände zwischen den Kreuzungspunkten 5
- tvl: natürliche Scheidenlänge
- tvl*: verkürzte Scheidenlänge

## Patentansprüche

1. Textiles, ein Netz mit Maschen umfassendes und einen nahtlosen Schlauch bildendes Implantat zur Ummantelung zumindest eines Teils eines Organs (K), wobei das Netz knotenlos, insbesondere gewirkt, gewebt oder gelegt ist und wobei der Schlauch zur radialen Anpassung an den Teil des Organs (K) kontrahierbar ist.

2. Implantat nach Anspruch 1 mit einer Maschenreihe aus nebeneinander liegenden Maschen, **gekennzeichnet durch** eine Erstreckung einer Maschenreihe in einem Winkel (β) gegenüber einer Längserstreckung (A) des schlauchförmigen Implantats.

3. Implantat nach Anspruch 1 oder 2, **gekennzeichnet durch** eine erste Gruppe (G1) von etwa parallel zueinander liegenden Strängen (3) aus mindestens zwei Fäden (3a; 3b) und eine zweite Gruppe (G2) von etwa parallel zueinander liegenden Strängen (4) aus mindestens zwei Fäden (4a; 4b), wobei die zweite Gruppe (G2) von Strängen (4) unter Ausbildung von Kreuzungsstellen (5) in einem Winkel (α) zu den Strängen (3) der ersten Gruppe (G1) verlaufen, und wobei die Stränge (3; 4) an den Kreuzungsstellen (5) miteinander verflochten sind, indem zumindest ein Faden (3a; 4a) des einen Strangs (3; 4) zwischen den Fäden (4a, 4b; 3a, 3b) des anderen Strangs (4; 3) verläuft.

4. Implantat nach Anspruch 1 oder 2, **dadurch gekennzeichnet, dass** die Stränge (3; 4) jeweils aus miteinander verdrillten Fäden (3a; 3b; 4a; 4b) bestehen und in einer Erstreckungsebene des abgewickelten Implantats (1) betrachtet durch Übereinanderliegen jeweils Verdrillungsstellen (6) und dazwischen liegende Schlaufen (7) bilden, und dass ein Faden (3a; 4a) des einen Strangs (3; 4) zwischen den Fäden (4a, 4b; 3a, 3b) einer Schlaufe (7) zwischen zwei Verdrillungsstellen (6) des anderen Strangs (4; 3) verläuft.

5. Implantat nach einem der obigen Ansprüche, **gekennzeichnet durch** unterschiedliche Maschenweiten.

6. Implantat nach einem der Ansprüche 3 bis 5, **dadurch gekennzeichnet, dass** die Stränge (3; 4) aus monofilen und/oder multifilen und/oder Mehrkomponenten-Fäden (3a, 3b; 4a, 4b) ausgebildet sind.

7. Implantat nach dem obigen Anspruch, **dadurch gekennzeichnet, dass** die monofilen oder multifilen oder Mehrkomponenten-Fäden der Stränge (3; 4) zumindest teilweise aus biokompatiblem resorbierbarem Material bestehen.

8. Implantat nach einem der obigen Ansprüche, **gekennzeichnet durch** eine Färbung der Stränge (3; 4).

9. Implantat nach einem der obigen Ansprüche, **gekennzeichnet durch** eine Fadenbeschichtung, insbesondere eine heilungsfördernde Fadenbeschichtung.

10. Implantat nach einem der obigen Ansprüche, **gekennzeichnet durch** daran angebrachtes chirurgisches Nahtmaterial oder benadeltes Nahtmaterial oder eine daran angebrachte Nadel als Fixierungsmittel.

11. Implantat nach einem der obigen Ansprüche, **dadurch gekennzeichnet, dass** es elastisches Fadenmaterial umfasst.

12. Implantat nach einem der obigen Ansprüche, **gekennzeichnet durch** Mittel zur Ausbildung eines Formgedächtnisses, insbesondere in einer Komprimierungsrichtung des Implantats.

13. Textiles, ein knotenloses Netz mit Maschen umfassendes und einen nahtlosen Schlauch bildendes Implantat zur Ummantelung zumindest eines Teils eines Organs (K), das zur radialen Anpassung an den Teil des Organs (K) kontrahierbar ist, hergestellt durch Wirken, Weben oder Legen, insbesondere durch Klöppeln oder Flechten.

14. Textiles Implantat nach einem der obigen Ansprüche zur medizinischen Anwendung, bevorzugt zur Anwendung bei der Vorbeugung und/oder Behandlung eines Organvorfalls im Bereich der Vagina, besonders bevorzugt zur Vorbeugung und/oder Behandlung einer Beckenbodeninsuffizienz, insbesondere eines Level-IDefekts nach DeLancey, oder zur Anwendung als zumindest teilweise resorbierbare Organumhüllung, bevorzugt als vollresorbierbare Organumhüllung, beispielsweise der Leber, der Niere und/oder der Milz.

15. Textiles Implantat nach einem der obigen Ansprüche zur Anwendung in einem chirurgischen, therapeutischen oder diagnostischen Verfahren, bevorzugt zur Anwendung bei einer Zervicosacropexie, bevorzugt nach einer suprazervicalen Hysterektomie, oder zur operativen Anwendung bei einer Sacrozervico-/-kolpopexie, bevorzugt bei einer totalen oder einer suprazervicalen Hysterektomie, insbesondere zur operativen Anwendung bei einer Sacrokolpopexie.
